# EUROPEAN PATENT APPLICATION

(11) **EP 3 391 891 A2**
(43) Date of publication of application: **24.10.2018**
(21) Application number: 17202129.7
(22) Date of filing: 08.06.2012
(51) Int. Cl.: A61K 33/00, A61M 16/12, A61P 9/12

(54) **PRESSURIZED VESSEL OF NITRIC OXIDE (NO)**

(30) Priority: 10.06.2011 US 201161495950 P
(62) Divisional of application: 12796066.4
(71) Applicant: Geno LLC, Cocoa, FL 32926 (US)
(72) Inventor: FINE, David.H., Cocoa Beach, FL 32932 (US); JOHNSON, Bryan, Orlando, FL 32833 (US)
(74) Representative: D Young & Co LLP

(57) **Abstract**

A pharmaceutical product can include a pressure vessel containing a pressurized gas including at least about 1% nitric oxide.

## Description

### CLAIM OF PRIORITY

This application claims the benefit of prior U.S. Provisional Application No. 61/495,950 filed on June 10, 2011.

### TECHNICAL FIELD

The invention relates to systems and methods for the storage and delivery of a gas including at least 1% nitric oxide.

### BACKGROUND

Nitric oxide (NO), also known as nitrosyl radical, is a free radical that is an important signalling molecule. For example, NO can cause smooth muscles in blood vessels to relax, thereby resulting in vasodilation and increased blood flow through the blood vessel. These effects can be limited to small biological regions since NO can be highly reactive with a lifetime of a few seconds and can be quickly metabolized in the body.

Some disorders or physiological conditions can be mediated by inhalation of nitric oxide. The use of low concentrations of inhaled nitric oxide can prevent, reverse, or limit the progression of disorders which can include, but are not limited to, acute pulmonary vasoconstriction, traumatic injury, aspiration or inhalation injury, fat embolism in the lung, acidosis, inflammation of the lung, adult respiratory distress syndrome, acute pulmonary edema, acute mountain sickness, post cardiac surgery acute pulmonary hypertension, persistent pulmonary hypertension of a newborn, perinatal aspiration syndrome, haline membrane disease, acute pulmonary thromboembolism, heparin-protamine reactions, sepsis, asthma and status asthmaticus or hypoxia. Nitric oxide can also be used to treat chronic pulmonary hypertension, bronchopulmonary dysplasia, chronic pulmonary thromboembolism and idiopathic or primary pulmonary hypertension or chronic hypoxia,

Generally, nitric oxide can be inhaled or otherwise delivered to the individual's lungs. Providing a therapeutic dose of NO could treat a patient suffering from a disorder or physiological condition that can be mediated by inhalation of NO or supplement or minimize the need for traditional treatments in such disorders or physiological conditions. Typically, the NO gas can be supplied in a bottled gaseous form diluted in nitrogen gas (N₂). Great care should be taken to prevent the presence of even trace amounts of oxygen (O₂) in the tank of NO gas because the NO, in the presence of O₂, can be oxidized to nitrogen dioxide (NO₂). Unlike NO, the part per million levels of NO₂ gas can be highly toxic if inhaled and can form nitric and nitrous acid in the lungs.

### SUMMARY

In one aspect, a pressure vessel can include a pressurized gas.

In some embodiments, a pressure vessel can be any container designed to hold a gas or liquid at a pressure that is substantially different than standard atmospheric pressure, for example, about 14.7 psi. For example, a pressure vessel can be designed to hold gases or liquids at a pressure of at least 20 psi, at least 100 psi, at least 500 psi, at least 700 psi, at least 1000 psi, at least 1500 psi, at least 2000 psi, at least 2500 psi or at least 3000 psi. In some embodiments, pressure can be determined at room temperature, for example, 20°C.

In some embodiments, a pressure vessel can hold a volume of at most about 20 L, at most about 10 L, at most about 5 L, at most about 1 L, at most about 500 mL, at most about 250 mL, at most about 100 mL, at most about 50 mL, at most about 25 mL, at most about 10 mL, at most about 5 mL, at most about 3 mL, at most about 2 mL or at most about 1 mL. In some embodiments, a pressure vessel can hold of volume of at least about 20 L, at least about 10 L, at least about 5 L, at least about 1 L, at least about 500 mL, at least about 250 mL, at least about 100 mL, at least about 50 mL, at least about 25 mL, at least about 10 mL, at least about 5 mL, at least about 3 mL, at least about 2 mL or at least about 1 mL.

In some embodiments, a pressurized gas can be a gas capable of remaining a gas at a pressure of at least 20 psi, at least 100 psi, at least 500 psi, at least 700 psi, at least 1000 psi, at least 1500 psi, at least 2000 psi, at least 2500 psi or at least 3000 psi. In some embodiments, pressure can be determined at room temperature, for example, 20°C.

In some embodiments, a pressurized gas can be contained in the pressure vessel at a pressure of at least 20 psi, at least 100 psi, at least 500 psi, at least 700 psi, at least 1000 psi, at least 1500 psi, at least 2000 psi, at least 2500 psi or at least 3000 psi. In some embodiments, pressure can be determined at room temperature, for example, 20°C.

In some embodiments, a pressurized gas can include at least 5% nitric oxide, at least about 25% nitric oxide, at least about 50% nitric oxide, at least about 75% nitric oxide, at least about 90% nitric oxide. In some embodiments, a pressurized gas can include at least about 4% and at most about 100% nitric oxide.

In some embodiments, a pressurized gas can include one or more inert gases. For example, a pressurized gas can include nitrogen (N₂). In some embodiments, the pressurized gas can consist essentially of nitric oxide.

In some embodiments, a pressurized gas can have a volume of at most about 2000L, at most about 1500 L, at most about 1000 L, at most about 800 L, at most about 500 L, at most about 250 L, at most about 100 L, at most about 50 L, at most about 25 L, at most about 20 L, at most about 10 L, at most about 5 L, at most about 2 L, at most about 1 L, at most about 500 mL, at most about 250 mL or at most about 100 mL at standard temperature and pressure, for example, at 20°C and about 14.7 psi. In some embodiments, a pressurized gas can have a volume of at least about at least about 100 L, at least about 50 L, at least about 25 L, at least about 20 L, at least about 10 L, at least about 5 L, at least about 2 L, at least about 1 L, at least about 500 mL, at least about 250 mL or at least about 100 mL at standard temperature and pressure, for example, at 20°C and about 14.7 psi.

In one aspect, a pharmaceutical product can include a pressure vessel containing a pressurized gas. In some embodiments, a pharmaceutical product can be used as a medicine or drug. In some embodiments, a pharmaceutical product can be a product that can be used for therapeutic treatment of an animal. Preferably, the animal is a mammal, most preferably, a human. In some embodiments, the pressurized gas can be suitable for inhalation by an animal.

In another aspect, a method of manufacturing a pharmaceutical product can include filling a pressure vessel with a pressurized gas.

In another aspect, a method of storing nitric oxide can include filling a pressure vessel with a pressurized gas.

In another aspect, a system can be a delivery system.

In some embodiments, a delivery system can include at least one tube and hardware required for connecting the elements of a delivery system. One tube can be used to connect or couple elements of a delivery system.

In some embodiments, a delivery system can include a first end. In some embodiments, a first end can be configured to be coupled to a pressure vessel. A first end can be directly or indirectly coupled to a pressure vessel.

In some embodiments, a delivery system can include a second end. In some embodiments, a delivery system can be configured to communicate a gas flow to a second end of a delivery system. A gas flow can include nitric oxide. In some embodiments, a second end of a delivery system can be coupled to a patient interface. A patient interface can include a mouth piece, nasal cannula, face mask, or fully-sealed face mask. In some embodiments, a second end of a delivery system can be coupled to a ventilator.

In some embodiments, a delivery system can include a coupler. A coupler can be configured to couple to an air source to provide air into a delivery system. Air from an air source can mix in a delivery system with a gas in a gas flow. This can result in a gas flow including at least 0.01% nitrogen dioxide, at least 0.05% nitrogen dioxide or at least 0.10% nitrogen dioxide.

In some embodiments, a delivery system can include an air source. In some embodiments, an air source can be configured to provide air into a delivery system. An air source can include an air pump or a hospital air supply. An air source can include an opening in a delivery system, such that the opening permits access to a volume of air. Air from an air source can mix in a delivery system with a gas in a gas flow. Air from an air source can mix in a delivery system with a gas in a gas flow. A gas flow can include at least 1% nitric oxide, at least 5% nitric oxide, at least about 25% nitric oxide, at least about 50% nitric oxide, at least about 75% nitric oxide, at least about 90% nitric oxide. In some embodiments, a gas flow can include at least about 4% and at most about 100% nitric oxide.

In some embodiments, a delivery system can include a coupler between the first end of the delivery system and the second end of the delivery system. A coupler can be configured to couple to an inert gas source.

In some embodiments, a delivery system can include an inert gas source. In some embodiments, an inert gas source can be between a first end of a delivery system and a second end of a delivery system.

An inert gas source can be configured to provide an inert gas into a delivery system. An inert gas from an inert gas source can mix in a delivery system with a gas in a gas flow, which can result in a dilution of the nitric oxide concentration in the gas flow. A gas flow can include at least 1% nitric oxide, at least 5% nitric oxide, at least about 25% nitric oxide, at least about 50% nitric oxide, at least about 75% nitric oxide, at least about 90% nitric oxide. In some embodiments, a gas flow can include at least about 4% and at most about 100% nitric oxide. In some embodiments, an inert gas source is a nitrogen source and an inert gas is nitrogen.

In some embodiments, a delivery system can include a reducing agent. In some embodiments, a reducing agent can include a hydroquinone, glutathione, and/or one or more reduced metal salts such as Fe(II), Mo(VI), NaI, Ti(III) or Cr(III), a thiol, or NO₂. A reducing agent can be an antioxidant. An antioxidant can be an aqueous solution of an antioxidant. An antioxidant can include ascorbic acid, alpha tocopherol, and/or gamma tocopherol. Any appropriate antioxidant can be used depending on the activities and properties as determined by a person of skill in the art. The antioxidant can be used dry or wet. A combination of reducing agents can be used.

In some embodiments, a reducing agent can be in a delivery system between a coupler and a second end. In some embodiments, a reducing agent can be in a delivery system between an air source and a second end. A reducing agent can react with nitrogen dioxide in a gas flow and can convert nitrogen dioxide to nitric oxide.

In some embodiments, a reducing agent can be included on a surface active material. In some embodiments, a reducing agent can be coated on the surface active material. In some embodiments, a surface active material can be in a cartridge configured to receive a gas flow including nitrogen dioxide via an inlet and to communicate the gas flow including nitrogen dioxide through the surface-active material to an outlet.

In some embodiments, a delivery system can be free of electronics. Electronics can be any device or portion of a device which requires electricity to operate.

In some embodiments, a delivery system can be free of a heating device. A heating device can include a heating element, a hot water bath, a heating mantle, heating wire or heating well. A heating element can include a simple flexible circuit board with the wires etched onto the surface.

In some embodiments, a delivery system can include a gas regulator coupled to a pressure vessel. A gas regulator can be any device which can alter the pressure of a gas. For example, a gas can enter a gas regulator at a high pressure and exit a gas regulator at a lower pressure, A gas regulator can be any device that can alter the rate at which a gas passes through the gas regulator, preferably a gas regulator can alter the rate to zero (i.e. stop the gas flow). A gas regulator can be directly or indirectly coupled to a pressure vessel.

In some embodiments, a delivery system can include a restrictor. In some embodiments, a restrictor can include a first end and a second end. In some embodiments, a restrictor can include a length corresponding to a distance between a first end of the restrictor and a second end of the restrictor, In some embodiments, a restrictor can be a gas regulator (i.e. an amount of gas flow released can be regulated by a restrictor) or a gas regulator can include a restrictor. A restrictor can be indirectly or directly coupled to a pressure vessel.

In some embodiments, a restrictor can include an orifice, a valve or a tube, A tube can include an internal diameter. In some embodiments, a tube can be a capillary tube, more specifically, a quartz capillary tube. In some embodiments, a length of the restrictor can be at least about 0,1 inch, at least about 0.25 inch or at least about 0.5 inch; the length can be at most about 4 inches, at most about 2 inches, at most about 1 inch, or at most about 0.5 inch. In some embodiments, the internal diameter of the restrictor can be at least about 0,001, at least about 0.005 microns or at least about 0.010; the internal diameter can be at most about 0.100 microns, at most about 0.050 microns, at most about 0.025 microns, or at most about 0.010 microns.

In some embodiments, a delivery system can include a pressure vessel.

In another aspect, a method can be a method for delivering nitric oxide. (X2)

In some embodiments, a method for delivering nitric oxide can include releasing a gas flow including nitric oxide from a pressure vessel into a delivery system, for example, a first end of a delivery system.

In some embodiments, a pressurized gas, which can become a gas flow once released, can be contained in the pressure vessel at a pressure of at least 20 psi, at least 100 psi, at least 500 psi, at least 700 psi, at least 1000 psi, at least 1500 psi, at least 2000 psi, at least 2500 psi or at least 3000 psi. In some embodiments, pressure can be determined at room temperature, for example, 20°C.

In some embodiments, a pressurized gas, which can become a gas flow once released, can have a volume of at most about 2000L, at most about 1500 L, at most about 1000 L, at most about 800 L, at most about 500 L, at most about 250 L, at most about 100 L, at most about 50 L, at most about 25 L, at most about 20 L, at most about 10 L, at most about 5 L, at most about 2 L, at most about 1 L, at most about 500 mL, at most about 250 mL or at most about 100 mL at standard temperature and pressure, for example, at 20°C and about 14.7 psi. In some embodiments, a pressurized gas can have a volume of at least about at least about 100 L, at least about 50 L, at least about 25 L, at least about 20 L, at least about 10 L, at least about 5 L, at least about 2 L, at least about 1 L, at least about 500 mL, at least about 250 mL or at least about 100 mL at standard temperature and pressure, for example, at 20°C and about 14.7 psi.

In some embodiments, a gas flow released from a pressurized vessel can include at least 5% nitric oxide, at least about 25% nitric oxide, at least about 50% nitric oxide, at least about 75% nitric oxide, at least about 90% nitric oxide. In some embodiments, a gas flow can include at least about 4% and at most about 100% nitric oxide.

In some embodiments, a gas flow released from a pressurized vessel can include one or more inert gases. For example, a pressurized gas can include nitrogen (N₂).

In some embodiments, a gas flow released from a pressurized vessel can consist essentially of nitric oxide.

In some embodiments, a method can include regulating an amount of the gas flow released from a pressure vessel. In some embodiments, an amount of gas flow released can be regulated by a gas regulator.

In some embodiments, a method can include providing air into a delivery system from an air source. An air source can include an air pump. An air source can include an opening in a delivery system, such that the opening permits access to a volume of air.

In some embodiments, a method can include mixing a gas flow with air in a delivery system such that, subsequent to mixing, the gas flow can include at least 0.01%, at least 0.1%, at least 1%, at least 5%, at least 10%, at least 25%, at least 50%, at least 75%, at least 90% or about 100% nitrogen dioxide.

In some embodiments, a method can include providing an inert gas into a delivery system from an inert gas source. In some embodiments, an inert gas is nitrogen and an inert gas source is a nitrogen source. A nitrogen source can include a gas bottle or liquid nitrogen.

In some embodiments, a method can include diluting a gas flow with an inert gas in a delivery system.

In some embodiments, a method can include converting nitrogen dioxide in a gas flow into nitric oxide.

In some embodiments, converting nitrogen dioxide in a gas flow into nitric oxide can include contacting a gas flow with a reducing agent. A reducing agent can react with nitrogen dioxide in a gas flow and can convert the nitrogen dioxide to nitric oxide.

In some embodiments, a method can include passing a gas flow out of a second end of a delivery system.

In some embodiments, a method can include delivering nitric oxide to a patient. In some embodiments, nitric oxide delivered to the patient is at a concentration of at least about 1 ppm, at least about 5 ppm, at least about 10 ppm, at least about 15 ppm or at least about 20 ppm.

Other features, objects, and advantages will be apparent from the description,

Other features, objects, and advantages will be apparent from the description, drawings, and claims.

### BRIEF DESCRIPTION OF THE DRAWINGS

FIG. 1 is a drawing depicting a conventional nitric oxide delivery platform.
FIG. 2 is a drawing depicting a nitric oxide delivery platform.
FIG. 3 is a drawing depicting a system to deliver nitric oxide.
FIG. 4 is a drawing depicting a cartridge.
FIG. 5 is a drawing depicting a cartridge.
FIG. 6 is a drawing depicting a cap of a cartridge.
FIG. 7 is a drawing depicting a system for delivering nitric oxide.

### DETAILED DESCRIPTION

When delivering nitric oxide (NO) for therapeutic use to a mammal, it can be important to avoid delivery of nitrogen dioxide (NO₂) to the mammal. Nitrogen dioxide (NO₂) can be formed by the oxidation of nitric oxide (NO) with oxygen (O₂). The rate of formation of nitrogen dioxide (NO₂) can be proportional to the oxygen (O₂) concentration multiplied by the square of the nitric oxide (NO) concentration. Additionally, nitric oxide can form nitrogen dioxide at increased concentrations.

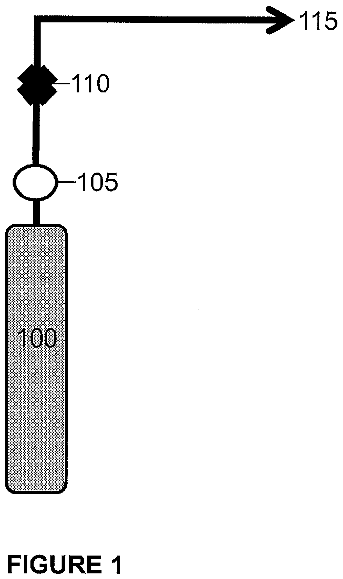

Platforms for delivering nitric oxide currently exist. For example, the standard platform in use can include a gas bottle 100 which contains 800 ppm NO in nitrogen (N₂) (FIG. 1). The nitric oxide/nitrogen gas can be released from the gas bottle 100 and the pressure and rate of the gas can be controlled using a gas regulator 105 and/or a valve 110. Using a gas bottle platform, the NO output 115 can be defined by the nitrogen dioxide concentration in the gas bottle 100 and cannot be varied by the user. For example, if the gas bottle contained 80 ppm of NO₂ in air or oxygen, then the output can be 80 ppm of NO₂ in air or oxygen. The gas can be supplied, typically, at a pressure of 2000 psi or greater. Typically, a gas bottle includes at least 99.9% N₂. A gas bottle platform can work well, but can be large, heavy and cumbersome because the platform can include a heavy aluminum or steel gas pressure cylinder, a gas regulator and a flow controller.

Examples of commercially available platforms are manufactured by Ikaria, two of which are the INOvent and the INOmax DS. Both of these systems use gas bottles of NO diluted in nitrogen (N₂), which is then mixed with oxygen enriched air to provide the inhaled NO gas. Both of these systems are designed to work with a ventilator in an intensive care setting in a hospital. These platforms are not suitable for ambulatory or home use.

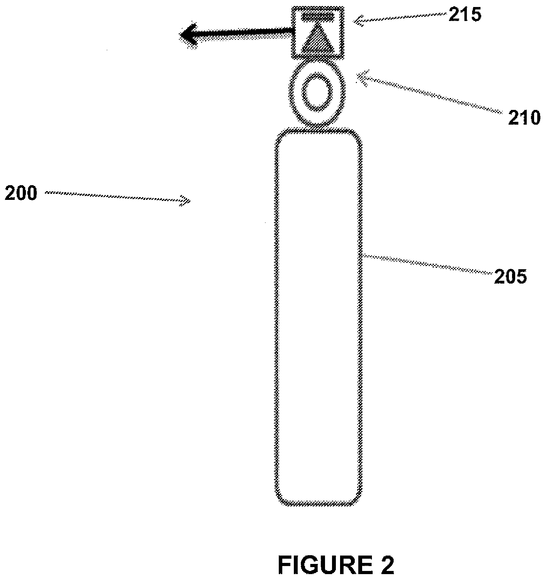

As another example, a platform can be a standalone gas bottle platform, as shown in FIG. 2. A gas bottle platform 200 can include a gas bottle 205, a gas regulator 210 and a GeNO cartridge 215. The output from the gas cylinder can be delivered to a GeNO cartridge, where one of the oxygen atoms in the NO₂ is stripped out by a reducing agent, for example, ascorbic acid, to generate ultra pure NO. The GeNO cartridge is described in greater detail below and in U.S. Patent Application Nos. 12/500, 929, 12/541,144, 12/619,959 and 12/951,811, and U.S. Patent No. 7,560,076, each of which is incorporated by reference in its entirety. This platform has been cleared by FDA for use in two clinical trials with human patients.

Another variation for delivering NO can be to start with a NO₂ gas concentration of up to 2,000 ppm in air or oxygen and dilute it down to 80 ppm of NO₂. This set up can be even more complex in that it can require precision mass flow controllers and meters in order to get a stable mixing ratio,

As mentioned above, the disadvantage of the gas bottle platform can be that the platform can be large and heavy. The platform can also be inconvenient to use for chronic treatment as an ambulatory platform. Gas bottles can also be cumbersome when used in a confined space such as in an Intensive Care Unit, in a hospital or in a home. In addition, the gas bottles need to be tied down to prevent them from falling over and causing physical injury. Also, the regulator can break off in a fall, and the sudden venting of gas through the opening can cause the heavy bottle to become a projectile, which can penetrate numerous walls and cause injury or death.
Therefore, there is a need for a nitric oxide delivery platform, which can includes a nitric oxide source which is small and portable for use in an ambulatory or home setting.

As one solution, a system can include a permeation tube or permeation cell to provide the source of NO₂. For example, the NO₂ source can be liquid dinitrogen tetroxide (N₂O₄). This approach has been shown to work well. This approach has been described in U.S. Patent Application No. 12/563,662, which is incorporated by reference in its entirety. N₂O₄ can vaporize to produce NO₂, and the process can be reversible. Using a permeation tube, air can be allowed to flow around the permeation tube, where it can mix with the NO₂ that diffuses through the tube, providing a stable mixture of NO₂ in air. The concentration of the NO₂ can be controlled by a number of factors including, for example, the temperature of the tube and the volume of the air flow. However, storing a permeation tube can be a problem. For instance, if NO₂ is in contact with the permeation tube polymer, the storage should be below -11 °C in order to keep the NO₂ frozen, which can prevent loss of NO₂. One solution is to build a separate storage chamber for the permeation tube, which can be connected to the storage tube by a simple valve. This device can be stored at room temperature without loss of NO₂, and it can easily be activated by connecting the reservoir to the permeation tube. The combined storage vessel and permeation tube can work well, but it can have one major disadvantage, Stabilization of a permeation tube can take a long time when the NO₂ is stored in a reservoir and then suddenly opened to the permeation tube. The time to stabilize can be several days. Pre-saturating the permeation tube with NO₂ first can speed up the stabilization, but this may not work well with long term storage of months or years.

As another solution, a reservoir assembly can be utilized. A system for deliverying a therapeutic amount of nitric oxide can include a reservoir assembly, a gas supply, and a delivery conduit. The delivery conduit can include at least one GeNO cartridge. This approach has been described in U.S. Patent Application No. 13/094,535, which is incorporated by reference in its entirety.

These solutions require the use of liquid nitrogen dioxide. Liquid nitrogen dioxide can be more difficult to use due to various governmental regulations, including shipping regulations. Additionally, vaporizing liquid nitrogen can require the use of a heating device and other electronics. Therefore, while smaller platforms are being developed, there remains a need for small platforms that use nitric oxide gas instead of liquid nitrogen dioxide.

A pressure vessel can be used to store or deliver nitric oxide. The vessel can include pressurized gas, for example, a gas including nitric oxide. A pressure vessel can be part of a pharmaceutical product (i.e, a medicine or drug). A pharmaceutical product can be a product that can be used for therapeutic treatment of an animal. Preferably, the animal is a mammal, most preferably, a human.

A pressure vessel can be any container designed to hold gases or liquids at a pressure that is substantially different than standard atmospheric pressure (e.g. approximately 14.7 psi). Specifically, a pressure vessel can be any container designed to hold gases or liquids at a pressure that is substantially greater than standard atmospheric pressure. For example, a pressure vessel can be designed to hold gases or liquids at a pressure of at least 20 psi, at least 100 psi, at least 500 psi, at least 700 psi, at least 1000 psi, at least 1500 psi, at least 2000 psi, at least 2500 psi or at least 3000 psi. Pressure can be determined at standard temperature, for example, 20°C.

A pressure vessel can be relatively large, for use in a hospital setting, for example, or relatively small, for use in an ambulatory or home setting, for example. A vessel can hold a volume of at most about 20 L, at most about 10 L, at most about 5 L, at most about 1 L, at most about 500 mL, at most about 250 mL, at most about 100 mL, at most about 50 mL, at most about 25 mL, at most about 10 mL, at most about 5 mL, at most about 3 mL, at most about 2 mL or at most about 1 mL. A pressure vessel can hold of volume of at least about 20 L, at least about 10 L, at least about 5 L, at least about 1 L, at least about 500 mL, at least about 250 mL, at least about 100 mL, at least about 50 mL, at least about 25 mL, at least about 10 mL, at least about 5 mL, at least about 3 mL, at least about 2 mL or at least about 1 mL.

A pressurized gas can be a gas capable of remaining a gas at a pressure of at least 20 psi, at least 100 psi, at least 500 psi, at least 700 psi, at least 1000 psi, at least 1500 psi, at least 2000 psi, at least 2500 psi or at least 3000 psi. Pressure can be determined at room temperature, for example, 20°C. In some cases, a pressurized gas can become a liquid or a pressurized liquid under enough pressure.

A pressurized gas can be contained in the pressure vessel at a pressure of at least 20 psi, at least 100 psi, at least 500 psi, at least 700 psi, at least 1000 psi, at least 1500 psi, at least 2000 psi, at least 2500 psi or at least 3000 psi. Pressure can be determined at room temperature. Room temperature can be between 15°C - 30°C or 20°C - 25°C. For example, room temperature can be about 20°C, 21°C, 22°C, 23°C, 24°C or 25°C.

A pressurized gas can include nitric oxide. In some cases, a pressurized gas can include at least 1% nitric oxide, at least 5% nitric oxide, at least about 25% nitric oxide, at least about 50% nitric oxide, at least about 75% nitric oxide, at least about 90% nitric oxide. A pressurized gas can include at least about 4% and at most about 100% nitric oxide. A pressurized gas can consist essentially of nitric oxide.

A pressurized gas can include one or more inert gases. For example, a pressurized gas can include nitrogen (N₂). Other inert gases can include, but are not limited to, helium, neon, argon, krypton, xenon, radon, and sulfur hexafluoride.

A pressurized gas can be suitable for inhalation by an animal. In other words, a pressurized gas and any resulting gas flow from the release of a pressurized gas should be pharmaceutical grade, for example, safe, pure and effective to administer to an animal. This means that the nitric oxide and inert gas (e.g. nitrogen) or the nitric oxide and air included in a pressurized gas or gas flow can be pharmaceutical grade.

A gas can be compressed when the gas is pressurized. The ratio of the gas at standard temperature and pressure to the pressurized gas can be at least 3:1, at least 5:1, at least 10:1, at least 20:1, at least 25: 1, at least 50:1, at least 100:1, at least 125:1 or at least 150:1. A pressurized gas can have a volume of at most about 2000L, at most about 1500 L, at most about 1000 L, at most about 800 L, at most about 500 L, at most about 250 L, at most about 100 L, at most about 50 L, at most about 25 L, at most about 20 L, at most about 10 L, at most about 5 L, at most about 2 L, at most about 1 L, at most about 500 mL, at most about 250 mL or at most about 100 mL at standard temperature and pressure, for example, at 20°C and about 14.7 psi. A pressurized gas can have a volume of at least about at least about 100 L, at least about 50 L, at least about 25 L, at least about 20 L, at least about 10 L, at least about 5 L, at least about 2 L, at least about 1 L, at least about 500 mL, at least about 250 mL or at least about 100 mL at standard temperature and pressure, for example, at 20°C and about 14.7 psi.

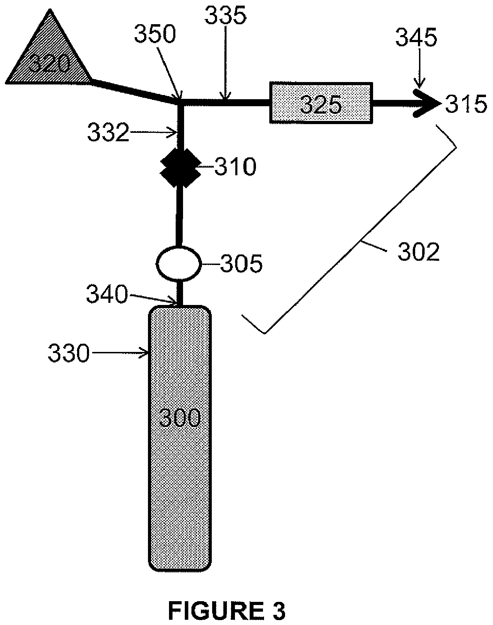

A vessel (or pharmaceutical product including a vessel) can be used with a system to deliver nitric oxide. A non-limiting embodiment of the system is shown in FIG. 3. A vessel 300 can be coupled to a delivery system 302. A delivery system 302 can include a first end 340, which can be coupled to a vessel 300 (e.g. a pressure vessel). The vessel 300 can contain gas including nitric oxide. The vessel 300 can include a higher percentage of nitric oxide compared to gas bottles currently in use, which include 0.08% nitric oxide in nitrogen. The vessel 300 can include at least 1% nitric oxide and up to 100% nitric oxide. The delivery system 302 can include tubing, reservoirs and other elements, which communicate gas released from a vessel to a second end 345 of the delivery system 302. The delivery system 302 can include or can be coupled (coupler 350) to an air source 320 between the first end 340 of the delivery system 302 and the second end 345 of the delivery system 302. An air source 320 can provide air into the delivery system 302. An air source can include an air pump or an air source can include an opening in a delivery system, such that the opening permits access to a volume of air.

Pressurized gas 330 from the pressure vessel 300 can be released, creating a gas flow 332. The gas flow 332 including nitric oxide can be released through a regulator 305 and/or restrictor 310. The gas flow can traverse through the delivery system. As the gas flow travels past the air source 320, air from the air source can mix with gas from the gas flow. As the air and the gas mix, the nitric oxide in the gas flow can rapidly react with oxygen in the air. The reaction can create nitrogen dioxide. This can result in a gas flow 335 including nitrogen dioxide. A percentage, in some cases, a high percentage of the nitric oxide in the gas flow can be converted to nitrogen dioxide. For example, it can be possible to have substantially all of the nitric oxide convert to nitrogen dioxide, such that the gas flow can include about 0% nitric oxide and about 100% nitrogen dioxide. The nitrogen dioxide can be acceptable using this system.

As the air and gas flow mix, the nitric oxide in the gas flow can be diluted, For example, if the gas flow included 80% nitric oxide (i.e. 80,000,000 ppm), the final concentration of nitric oxide in the gas flow could be reduced, for example, to 0.08% (800 ppm). The dilution can be controlled by controlling the relative rates of the gas flow and the air and/or the relative amounts of the gas and the air, The gas flow including nitrogen dioxide can also be released through a regulator and/or restrictor following nitrogen dioxide formation.

The gas flow 335 can come in contact with a reducing agent 325 between the air source and the second end. The reducing agent can react with nitrogen dioxide in the gas flow and can convert the nitrogen dioxide to nitric oxide. The reducing agent can be an element in a GeNO cartridge, described in greater detail below. The gas flow 315 that is delivered can include nitric oxide and substantially no nitrogen dioxide, as the nitrogen dioxide can be completely converted to nitric oxide.

A vessel (or pharmaceutical product including a vessel) can be used in a delivery system. A delivery system can be used for the delivery nitric oxide. A delivery system can include at least one tube and hardware (i.e. clamps, connectors, valves, bends, etc.) required for connecting the elements of a delivery system. An at least one tube can be used to connect or couple elements of a delivery system together.

A delivery system can include a first end and a second end. A first end can be configured to be directly or indirectly coupled to a pressure vessel. A pressure vessel can include a pressurized gas, which can include at least 1% nitric oxide, as described above.

A delivery system can be configured to communicate a gas flow to a second end of a delivery system. A gas flow can include nitric oxide. A second end of a delivery system can be coupled to a patient interface, for example, a mouth piece, nasal cannula, face mask, or fully-sealed face mask. A second end of a delivery system can be coupled to a ventilator.

A delivery system can include a coupler. A coupler can be configured to couple to an air source to provide air into a delivery system.

A delivery system can include an air source, which can be configured to provide air into a delivery system.

An air source can include an air pump or a hospital air supply. An air source can include an opening in a delivery system, such that the opening permits access to a volume of air. Air from an air source can mix in a delivery system with a gas in a gas flow. For example, as the gas flow moves past an air source which can be an opening, negative pressure can be created in the delivery system at the opening. The negative pressure will pull air through the opening and into the delivery system, where it can mix with gas from the gas flow. Alternatively, an air pump can pump air into the delivery system.

Air from an air source can mix in a delivery system with a gas in a gas flow. The nitric oxide in the gas flow can react with the oxygen in the air to rapidly form nitrogen dioxide. This reaction can be expected. A percentage of the nitric oxide can convert to nitrogen dioxide. The percentage can be at least 50%, at least 75%, at least 90% or about 100%. The mixing of the air and the gas can result in a gas flow including at least 0.01%, at least 0.1%, at least 1%, at least 5%, at least 10%, at least 25%, at least 50%, at least 75%, at least 90% or about 100% nitrogen dioxide.

While current nitric oxide delivery platforms avoid the formation of nitrogen dioxide, the formation of nitrogen dioxide in the delivery system can be both expected and intended. That is because nitrogen dioxide can be eliminated from the gas flow prior to delivery using a reducing agent.

Alternatively, a coupler can be configured to couple to an inert gas source.

A delivery system can include an inert gas source, which can be between a first end of a delivery system and a second end of a delivery system,

An inert gas source can be configured to provide an inert gas into a delivery system. An inert gas from an inert gas source can mix in a delivery system with a gas in a gas flow, which can result in a dilution of the nitric oxide concentration in the gas flow. In some embodiments, an inert gas source is a nitrogen source and an inert gas is nitrogen.

A delivery system can include an inert gas source. An inert gas source can be between a first end of a delivery system and a second end of a delivery system. An inert gas source can be configured to provide an inert gas into a delivery system. An inert gas from an inert gas source can mix in a delivery system with a gas in a gas flow, which can result in a dilution of the nitric oxide concentration in the gas flow. An inert gas source can be a nitrogen source and an inert gas can be nitrogen.

A delivery system can include a reducing agent. A reducing agent can include a hydroquinone, glutathione, and/or one or more reduced metal salts such as Fe(II), Mo(VI), NaI, Ti(III) or Cr(III), a thiol, or NO₂⁻. A reducing agent can be an antioxidant. An antioxidant can be an aqueous solution of an antioxidant. An antioxidant can include ascorbic acid, alpha tocopherol, and/or gamma tocopherol. Any appropriate antioxidant can be used depending on the activities and properties as determined by a person of skill in the art. The antioxidant can be used dry or wet. A combination of reducing agents can be used.

A reducing agent can be in a delivery system between a coupler and a second end or between an air source and a second end. A reducing agent can be present in the system at any location after a location where nitrogen dioxide can be formed. A reducing agent can react with nitrogen dioxide in a gas flow and can convert nitrogen dioxide to nitric oxide.

A reducing agent can be included on a surface active material. A reducing agent can be coated on the surface active material. In preferred embodiments, a surface active material can be in a GeNO cartridge, which is a cartridge configured to receive a gas flow including nitrogen dioxide via an inlet and to communicate the gas flow including nitrogen dioxide through the surface-active material to an outlet.

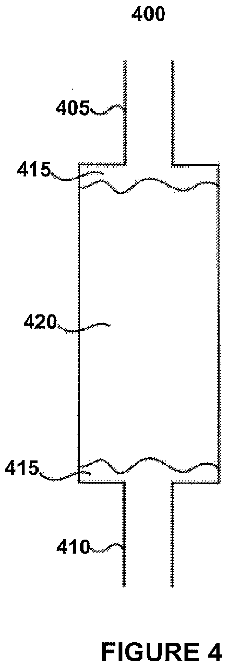

FIG. 4 illustrates a cartridge 400 for generating NO by converting NO₂ to NO. The cartridge 400, which may be referred to as a cartridge, a converter, a NO generation cartridge, a GENO cartridge, a GENO cylinder, GENO converter or Nitrosyl™ Primary Cartridge, can include an inlet 405 and an outlet 410. Screen and/or glass wool 415 can be located at the inlet 405 and/or the outlet 410. The remainder of the cartridge 400 can be filled with a surface-active material 420 that is soaked with a saturated solution including a reducing agent to coat the surface-active material. The saturated solution can be, for example, an antioxidant in water. The screen and/or glass wool 415 can also be soaked with the saturated solution before being inserted into the cartridge 400. The antioxidant can be ascorbic acid.

In a general process for converting NO₂ to NO, a gas flow (e.g. air flow) having NO₂ can be received through the inlet 405. The gas flow can be fluidly communicated to the outlet 410 through the surface-active material 420 coated with the aqueous reducing agent, e.g. antioxidant. As long as the surface-active material 420 remains moist and the reducing agent may not been used up in the conversion, the general process can be effective at converting NO₂ to NO at ambient temperature.

The inlet 405 also may receive the air flow having NO₂, for example, from source of NO₂. A source of NO₂ can include a pressurized bottle of NO₂, which also may be referred to as a tank of NO₂. The inlet 405 also may receive a gas flow with NO₂ in nitrogen, air, or oxygen. The conversion can occur over a wide concentration range. Experiments have been carried out at concentrations in a gas including from about 2 ppm NO₂ to 100 ppm NO₂, and even to over 1000 ppm NO₂. In one example, a cartridge that was approximately 6 inches long and had a diameter of 1.5-inches was packed with silica gel that had first been soaked in a saturated aqueous solution of ascorbic acid. The moist silica gel was prepared using ascorbic acid (i.e., vitamin C) designated as A.C.S reagent grade 99.1% pure from Aldrich Chemical Company and silica gel from Fischer Scientific International, Inc., designated as S8 32-1, 40 of Grade of 35 to 70 sized mesh. Other sizes of silica gel also can be effective. For example, silica gel having an eighth-inch diameter could also work.

The silica gel can be moistened with a saturated solution including a reducing agent. For example, a saturated solution of ascorbic acid in water; more specifically, the saturated solution can be a saturated solution that had been prepared by mixing 35% by weight ascorbic acid in water, stirring, and straining the water/ascorbic acid mixture through the silica gel, followed by draining. The conversion of NO₂ to NO can proceed well when the silica gel coated with ascorbic acid is moist, The conversion of NO₂ to NO may not proceed well in an aqueous solution of ascorbic acid alone.

The cartridge can be filled with the wet silica gel/reducing agent. For example, a cartridge filled with the wet silica gel/ascorbic acid was able to convert 1000 ppm of NO₂ in air to NO at a flow rate of 150 ml per minute, quantitatively, non-stop for over 12 days. A wide variety of flow rates and NO₂ concentrations have been successfully tested, ranging from only a few ml per minute to flow rates of up to 5,000 ml per minute. Any appropriate reducing agent that can convert NO₂ or N₂O₄ to NO can be used as determined by a person of skill in the art.

The antioxidant/surface-active material GENO cartridge may be used for inhalation therapy. In one such example, the GENO cartridge can be used as a NO₂ scrubber for NO inhalation therapy that delivers NO from a pressurized bottle source. The GENO cartridge can be used to remove any NO₂ that chemically forms during inhalation therapy. This GENO cartridge can be used to help ensure that no harmful levels of NO₂ are inadvertently inhaled by the patient.

Using the system as an inhaled NO drug delivery device, the NO₂ output in air or oxygen can be passed through a GeNO cartridge, which strips out one of the O atoms from the NO₂ to produce ultra pure NO.

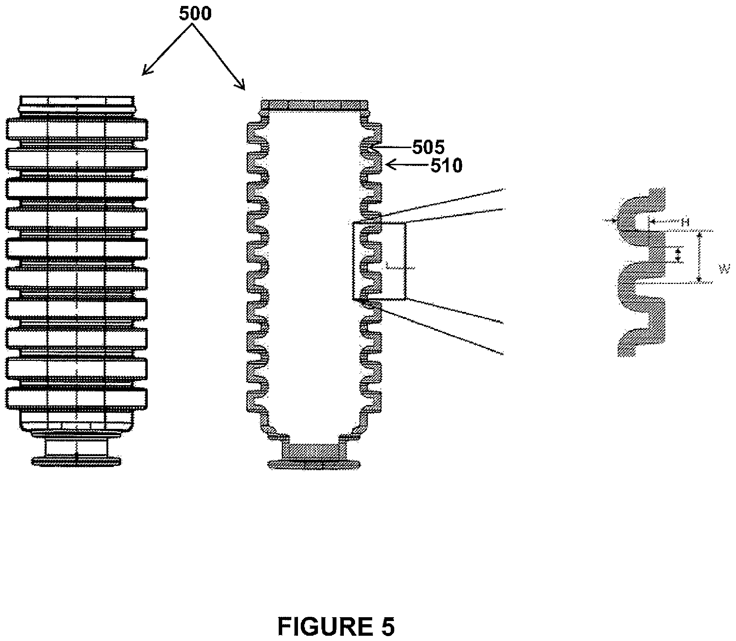

Referring to FIG. 5, the cartridges 500 can be blow-molded with internal ridges 505 and valleys 510. When packed with the surface active material and reducing agent (e.g, silica gel/ascorbic acid powder), the particles can tend to pack, leaving a small air gap at the top. If the cartridge was allowed to be vibrated on its side, the material could settle. If the tube had a smooth bore, the space above the powder could create a path that bypassed the GeNO converter. By having the ridges 505 and valleys 510, the powder can settle and the vapour cannot have a pathway that would bypass the chemistry reactor. The height of the ridge and its width can be determined by calculation and then confirmed experimentally.

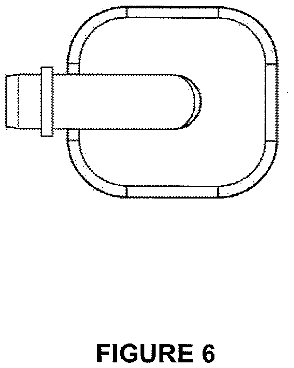

The cap for the cartridges can be molded from plastic (FIG. 6).

In order to allow the system to be used in a wider range of situations and/or locations, a delivery system can be free of electronics. Electronics can be any device or portion of a device which requires electricity to operate. Electronics can include computers, processors, wires, power cords, batteries, lights, alarms, etc.

A delivery system can be free of a heating device, A heating device can include a heating element, a hot water bath, a heating mantle, heating wire or heating well. A heating element can include a simple flexible circuit board with the wires etched onto the surface. In some situations, if heating is required, body heat can be used to raise the temperature of the delivery system or a portion of a delivery system.

A delivery system can include a gas regulator coupled to a pressure vessel. A gas regulator can be a restrictor. In some embodiments, an amount of gas flow released can be regulated by a gas regulator. A gas regulator can be any device which can alter the pressure of a gas. For example, a gas can enter a gas regulator at a high pressure and exit a gas regulator at a lower pressure. A gas regulator can be any device that can alter the rate at which a gas passes through the gas regulator, preferably a gas regulator can alter the rate to zero (i.e. stop the gas flow). A gas regulator can be directly or indirectly coupled to a pressure vessel. In some embodiments, an amount of gas flow released can be regulated by a restrictor. In other words, a gas regulator can include a restrictor. A restrictor can be indirectly or directly coupled to a pressure vessel.

A delivery system can include a restrictor. A restrictor can be any device which can limit the flow of gas from the vessel. A restrictor can require that there be enough pressure to force the gas through the restrictor. A restrictor can include an orifice, a valve or a tube. A tube can include an internal diameter. In some embodiments, a tube can be a capillary tube, more specifically, a quartz capillary tube.

A restrictor can have any dimension, so long as the total pressure drop across the restrictor can be appropriate for the flow of NO that is required. A restrictor can include a first end and a second end. A restrictor can include a length corresponding to a distance between a first end of the restrictor and a second end of the restrictor. In some embodiments, a length of the restrictor can be relatively long, for example, greater than 4 inches, greater than 6 inches, greater than 1 foot, greater than 2 feet, greater than 5 feet, greater than 10 feet or greater than 20 feet long. In some embodiments, a restrictor can be relatively short, for example, at least about 0.1 inch, at least about 0.25 inch or at least about 0.5 inch; the length can be at most about 4 inches, at most about 2 inches, at most about 1 inch, or at most about 0.5 inch. In some embodiments, the internal diameter of the restrictor can be relatively large, for example, greater than about 0.100 microns, greater than about 1 micron, greater than about 5 microns, greater than about 10 microns, greater than about 50 microns or greater than about 100 microns. In some embodiments, the internal diameter of the restrictor can be relatively small, for example, at least about 0.001, at least about 0.005 microns or at least about 0.010; the internal diameter can be at most about 0.100 microns, at most about 0.050 microns, at most about 0.025 microns, or at most about 0.010 microns.

The amount of material (e.g. nitrogen dioxide) that is forced through the restrictor at any temperature can be dependent upon the diameter of the restrictor, Thus, key design variables can be the temperature of the vessel and the diameter and length of the restriction in the top of the vessel.

A restrictor can be made of other materials known to those of skill in the art. The material should not react with or adsorb NO, N₂O₄ or NO₂.

In some embodiments, a delivery system can include a pressure vessel.

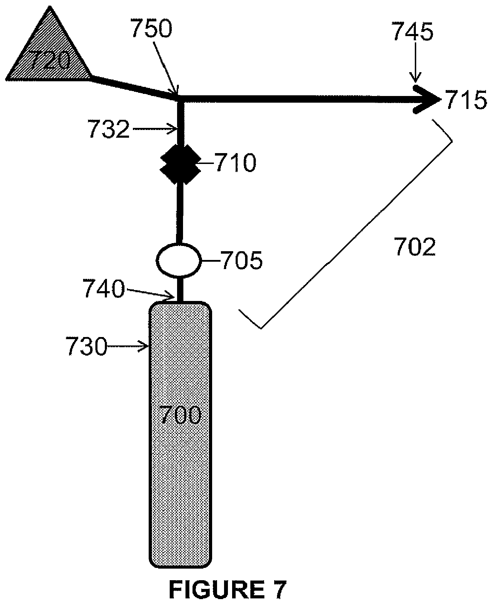

In another aspect, a vessel (or pharmaceutical product including a vessel) can be used with a system to deliver nitric oxide, which may not include air. A non-limiting embodiment of the system is shown in FIG. 7. A vessel 700 can be coupled to a delivery system 702. A delivery system 702 can include a first end 740, which can be coupled to a vessel 700 (e.g. a pressure vessel). The vessel 700 can contain gas including nitric oxide. The vessel 700 can include a higher percentage of nitric oxide compared to gas bottles currently in use, which include 0.08% nitric oxide in nitrogen. The vessel 700 can include at least 1% nitric oxide and up to 100% nitric oxide. The delivery system 702 can include tubing, reservoirs and other elements, which communicate gas released from a vessel to a second end 745 of the delivery system 702. The delivery system 702 can include or can be coupled (coupler 750) to an inert gas source 720 between the first end 740 of the delivery system 702 and the second end 745 of the delivery system 702. An inert gas source 740 can provide air into the delivery system 702. An inert gas source can include a gas bottle.

The inert gas can enter into the delivery system 702 and can mix with the gas including nitric oxide 732, resulting in a diluted gas flow 715 including a lower concentration of nitric oxide. In other words, gas flow 732 including a high concentration of nitric oxide can be mixed with an inert gas to produce a lower concentration nitric oxide, for example, 800 ppm NO.

This system can deliver similar concentrations of NO and N₂ as delivery platforms currently in use. However, by varying the mixing ratio (i.e. by adding an inert gas to dilute the nitric oxide), the NO concentration can be decreased to with greater accuracy at lower concentrations than can currently be achieved. Greater accuracy at low doses in the 0.1 to 5 ppm range can be important to allow for more precise weaning of a patient. Currently the turn down ratio is such that the accuracy at 2 ppm can be +- 50% or more. With this system, as with all of the GeNO designs, accuracies can be +-3%. Another advantage can be that the NO can be stored in relatively small vials, which can be stocked in a pharmacy.

The inert gas can be nitrogen gas, which can come from a supply of liquid N₂. Liquid N₂ with suitable controls or N₂ from a bench top N₂ generator (See Schmidlin-DBS AG, http://www.ddprocess.com/lab-gas-generator/mini-nitrogen-generator.pdf.) can be used.

A method can be a method for delivering nitric oxide. A method for delivering nitric oxide can include releasing a gas flow including nitric oxide from a pressure vessel into a delivery system. A first end of a delivery system can be coupled to a pressure vessel. A delivery system can be configured to communicate a gas flow to a second end of the delivery system. A method can include regulating an amount of the gas flow released from a pressure vessel. An amount of gas flow released can be regulated by a gas regulator. A pressurized gas can become a gas flow when it is released from a pressure vessel.

A gas flow released from a pressurized vessel can include at least 1% nitric oxide, at least 5% nitric oxide, at least about 25% nitric oxide, at least about 50% nitric oxide, at least about 75% nitric oxide, at least about 90% nitric oxide, A gas flow released from a pressurized vessel can include at least about 4% and at most about 100% nitric oxide.

A gas flow released from a pressurized vessel can include one or more inert gases. For example, a pressurized gas can include nitrogen (N₂). Other inert gases can include, but are not limited to, helium, neon, argon, krypton, xenon, radon, and sulfur hexafluoride. Alternatively, a gas flow released from a pressurized vessel can consist essentially of nitric oxide. A pressurized gas can become a gas flow once released.

A method can include providing air into a delivery system from an air source. An air source can include an air pump or a hospital air supply. An air source can include an opening in a delivery system, such that the opening permits access to a volume of air.

A method can include mixing a gas flow with air in a delivery system such that, subsequent to mixing, the gas flow can include at least 0.01%, at least 0.1%, at least 1%, at least 5%, at least 10%, at least 25%, at least 50%, at least 75%, at least 90% or about 100% nitrogen dioxide.

A method can include converting nitrogen dioxide in a gas flow into nitric oxide. Converting nitrogen dioxide in a gas flow into nitric oxide can include contacting a gas flow with a reducing agent. As described above, a reducing agent can react with nitrogen dioxide in a gas flow and can convert the nitrogen dioxide to nitric oxide.

A method can include providing an inert gas into a delivery system from an inert gas source. An inert gas is nitrogen and an inert gas source is a nitrogen source. A nitrogen source can include a gas bottle or liquid nitrogen.

A method can include diluting a gas flow with an inert gas in a delivery system. For example, a gas flow can be released from the pressure vessel including a high concentration of nitric oxide in a low concentration of inert gas. An inert gas can be added in the delivery system, which can dilute the concentration of nitric oxide, resulting in a gas flow with a low concentration of nitric oxide and a high concentration of inert gas. An inert gas can include more than one inert gas.

The ratio of the nitric oxide concentration in the gas released from the vessel to the gas after dilution can be at least 1:100, at least 1:500, at least 1:1000, at least 1:1250, at least 1:1500, at least 1:2000, at least 1:5000, at least 1:10,000, at least 1:25,000 or at least 1:50,000.

A method can include passing a gas flow out of a second end of a delivery system or passing a gas flow out of a patient interface coupled to a second end of a delivery system. The nitric oxide can be suitable for inhalation by an animal, preferably, a mammal. A patient interface can be directly or indirectly coupled to a second end of the delivery system. A patient interface can include a mouth piece, nasal cannula, face mask, or fully-sealed face mask. A ventilator can be directly or indirectly coupled to a second end of a delivery system.

A method can include delivering nitric oxide to a patient. Nitric oxide delivered to the patient is at a concentration of at least about 1 ppm, at least about 5 ppm, at least about 10 ppm, at least about 15 ppm or at least about 20 ppm.

Details of one or more embodiments are set forth in the accompanying drawings and description. Other features, objects, and advantages will be apparent from the description, drawings, and claims. Although a number of embodiments of the invention have been described, it will be understood that various modifications may be made without departing from the spirit and scope of the invention. It should also be understood that the appended drawings are not necessarily to scale, presenting a somewhat simplified representation of various features and basic principles of the invention.

Various preferred features and embodiments of the present invention will now be described with reference to the following numbered paragraphs (paras).
1. A pharmaceutical product, comprising:
   a pressure vessel containing a pressurized gas including at least about 1% nitric oxide.
2. The pharmaceutical product of para 1, wherein the pressurized gas is suitable for inhalation by an animal.
3. The pharmaceutical product of para 1 or 2, wherein the pressurized gas includes at least about 5% nitric oxide.
4. The pharmaceutical product of any one of paras 1-3, wherein the pressurized gas includes at least about 25% nitric oxide.
5. The pharmaceutical product of any one of paras 1-4, wherein the pressurized gas includes at least about 50% nitric oxide.
6. The pharmaceutical product of any one of paras 1-5, wherein the pressurized gas includes at least about 75% nitric oxide.
7. The pharmaceutical product of any one of paras 1-6, wherein the pressurized gas includes at least about 90% nitric oxide.
8. The pharmaceutical product of any one of paras 1-7, wherein the pressurized gas comprises at least about 4% and at most about 100% nitric oxide.
9. The pharmaceutical product of any one of paras 1-8, wherein the pressurized gas comprises one or more inert gases.
10. The pharmaceutical product of any one of paras 1-9, wherein the pressurized gas comprises nitrogen.
11. The pharmaceutical product of any one of paras 1-8, wherein the pressurized gas consists essentially of nitric oxide.
12. The pharmaceutical product of any one of paras 1-11, wherein the pressure vessel holds a volume of at most about 10 mL.
13. The pharmaceutical product of any one of paras 1-12, wherein the pressure vessel holds a volume of at most about 5 mL.
14. The pharmaceutical product of any one of paras 1-13, wherein the pressure vessel holds a volume of at most about 3 mL.
15. The pharmaceutical product of any one of paras 1-14, wherein the pressurized gas is contained in the pressure vessel at a pressure of at least 700 psi.
16. The pharmaceutical product of any one of paras 1-15, wherein the pressurized gas is contained in the pressure vessel at a pressure of at least 1500 psi.
17. The pharmaceutical product of any one of paras 1-16, wherein the pressurized gas has a volume of at most about 20 L at standard temperature and pressure.
18. The pharmaceutical product of any one of paras 1-17, wherein the pressurized gas has a volume of at most about 2 L at standard temperature and pressure.
19. The pharmaceutical product of any one of paras 1-18, wherein the pressurized gas has a volume of at most about 500 mL at standard temperature and pressure.
20. A delivery system, comprising:
   a first end configured to be coupled to a pressure vessel including a pressurized gas, wherein the pressurized gas includes at least 1% nitric oxide;
   a second end, wherein the delivery system is configured to communicate a gas flow including nitric oxide to a second end of the delivery system; and
   a coupler, wherein the coupler is configured to couple to an air source to provide air into the delivery system, such that air from the air source mixes in the delivery system with the gas in the gas flow resulting in a gas flow including at least 0.01% nitrogen dioxide; and
   a reducing agent between the coupler and the second end, wherein the reducing agent reacts with nitrogen dioxide in the gas flow and converts the nitrogen dioxide to nitric oxide.
21. The system of para 20, comprising an air source configured to provide air into the delivery system, such that air from the air source mixes in the delivery system with the gas in the gas flow resulting in a gas flow including at least 0.01% nitrogen dioxide.
22. The system of para 20 or 21, wherein the reducing agent is included on a surface active material.
23. The system of any one of paras 20-22, wherein the reducing agent is coated on the surface active material.
24. The system of any one of paras 20-23, wherein the surface active material is in a cartridge configured to receive the gas flow including nitrogen dioxide via an inlet and to communicate the gas flow including nitrogen dioxide through the surface-active material to an outlet.
25. The system of any one of paras 20-24, wherein the delivery system is free of electronics.
26. The system of any one of paras 20-25, wherein the delivery system is free of a heating device.
27. The system of any one of paras 20-26, wherein the delivery system includes a gas regulator coupled to the storage device.
28. The system of any one of paras 20-27, wherein the delivery system includes a restrictor.
29. The system of para 28, wherein the restrictor includes an orifice,
30. The system of para 28 or 29, wherein the restrictor includes a capillary tube.
31. The system of any one of paras 20-30, wherein the second end of the delivery system is coupled to a patient interface.
32. The system of any one of paras 20-31, wherein the second end of the delivery system is coupled to a ventilator.
33. The system of any one of paras 20-32, wherein the system includes a pressure vessel containing a pressurized gas including at least about 1% nitric oxide.
34. A method for delivering nitric oxide, comprising:
   releasing a gas flow including nitric oxide from a pressure vessel into a delivery system, wherein a first end of the delivery system is coupled to the pressure vessel and the delivery system is configured to communicate the gas flow to a second end of the delivery system;
   providing air into the delivery system from an air source;
   mixing the gas flow with the air in the delivery system such that, subsequent to mixing, the gas flow includes at least 0.01% nitrogen dioxide;
   converting the nitrogen dioxide in the gas flow into nitric oxide; and
   passing the gas flow out of the second end of the delivery system.
35. The method of para 34, wherein converting the nitrogen dioxide in the gas flow into nitric oxide includes contacting the gas flow with a reducing agent, wherein the reducing agent reacts with nitrogen dioxide in the gas flow and converts the nitrogen dioxide to nitric oxide.
36. The method of para 35, wherein the reducing agent is included on a surface active material.
37. The method of para 36, wherein the reducing agent is coated on the surface active material.
38. The method of para 36 or 37, wherein the surface active material is in a cartridge configured to receive the gas flow including nitrogen dioxide via an inlet and to communicate the gas flow including nitrogen dioxide through the surface-active material to an outlet.
39. The method of any one of paras 34-38, wherein the delivery system is free from electronics.
40. The method of any one of paras 34-39, wherein the delivery system is free of a heating device,
41. The method of any one of paras 34-40, wherein the method includes regulating an amount of the gas flow released from a pressure vessel.
42. The method of para 41, wherein the amount of gas flow released is regulated by a gas regulator, which is coupled to the pressure vessel.
43. The method of para 41 or 42, wherein the amount of gas flow released is regulated by a restrictor, which is coupled to the pressure vessel.
44. The method of para 43, wherein the restrictor includes an orifice.
45. The method of para 43 or 44, wherein the restrictor includes a capillary tube.
46. The method of any one of paras 34-45, wherein the nitric oxide is suitable for inhalation by a mammal.
47. The method of any one of paras 34-46, wherein a patient interface is coupled to the second end of the delivery system.
48. The method of any one of paras 34-47, wherein a ventilator is coupled to the second end of the delivery system.
49. The method of any one of paras 34-48, wherein the method includes delivering nitric oxide to a patient.
50. The method of para 49, wherein the nitric oxide delivered to the patient is at a concentration of at least about 1 ppm.
51. The method of para 49 or 50, wherein the nitric oxide delivered to the patient is at a concentration of at least about 5 ppm.
52. The method of any one of paras 49-51, wherein the nitric oxide delivered to the patient is at a concentration of at least about 10 ppm.
53. The method of any one of paras 49-52, wherein the nitric oxide delivered to the patient is at a concentration of at least about 15 ppm.
54. The method of any one of paras 49-53, wherein the nitric oxide delivered to the patient is at a concentration of at least about 20 ppm.
55. The method of any one of paras 34-54, wherein the gas flow released from the pressure vessel includes at least about 1% nitric oxide.
56. The method of any one of paras 34-55, wherein the gas flow released from the pressure vessel includes at least about 5% nitric oxide.
57. The method of any one of paras 34-56, wherein the gas flow released from the pressure vessel includes at least about 25% nitric oxide.
58. The method of any one of paras 34-57, wherein the gas flow released from the pressure vessel includes at least about 50% nitric oxide.
59. The method of any one of paras 34-58, wherein the gas flow released from the pressure vessel includes at least about 75% nitric oxide.
60. The method of any one of paras 34-59, wherein the gas flow released from the pressure vessel includes at least about 90% nitric oxide.
61. The method of any one of paras 34-60, wherein the gas flow released from the pressure vessel includes at least about 4% and at most about 100% nitric oxide.
62. The method of any one of paras 34-61, wherein the gas flow released from the pressure vessel includes one or more inert gases.
63. The method of any one of paras 34-62, wherein the gas flow released from the pressure vessel includes nitrogen.
64. The method of any one of paras 34-63, wherein the gas flow released from the pressure vessel consists essentially of nitric oxide.
65. The method of any one of paras 34-64, wherein the pressure vessel holds a volume of at most about 10 mL.
66. The method of any one of paras 34-65, wherein the pressure vessel holds a volume of at most about 5 mL.
67. The method of any one of paras 34-66, wherein the pressure vessel holds a volume of at most about 3 mL.
68. A method of storing nitric oxide, comprising:
   filling a pressure vessel with a pressurized gas including nitric oxide, wherein the pressurized gas is suitable for inhalation by a mammal, and wherein the pressure vessel holds volume of less than 5 L, the pressurized gas is stored at a pressure of at least about 1500 psi and the pressurized gas has a volume of at most about 800 L at standard temperature and pressure.
69. A method of manufacturing a pharmaceutical product, comprising:
   filling a pressure vessel with a pressurized gas including nitric oxide, wherein the pressurized gas is suitable for inhalation by a mammal, and wherein the pressure vessel holds volume of less than 5 L, the pressurized gas is stored at a pressure of at least about 1500 psi and the pressurized gas has a volume of at most about 800 L at standard temperature and pressure.
70. A delivery system, comprising:
   a first end configured to be coupled to a pressure vessel including a pressurized gas, wherein the pressurized gas includes at least 1% nitric oxide;
   a second end, wherein the delivery system is configured to communicate a gas flow including nitric oxide to a second end of the delivery system; and
   a coupler between the first end of the delivery system and the second end of the delivery system, the coupler being configured to couple to an inert gas source, wherein the inert gas source is configured to provide an inert gas into the delivery system such that the inert gas dilutes the nitric oxide concentration in the gas flow,
71. The system of para 70, comprising an inert gas source.
72. The system of para 70 or 71, wherein the inert gas source is a nitrogen source and the inert gas is nitrogen.
73. The system of any one of paras 70-72, wherein the delivery system is free of electronics.
74. The system of any one of paras 70-73, wherein the delivery system is free of a heating device.
75. The system of any one of paras 70-74, wherein the delivery system includes a gas regulator coupled to the storage device,
76. The system of any one of paras 70-75, wherein the delivery system includes a restrictor.
77. The system of para 76, wherein the restrictor includes an orifice.
78. The system of para 76 or 77, wherein the restrictor includes a capillary tube.
79. The system of any one of paras 70-78, wherein the second end of the delivery system is coupled to a patient interface.
80. The system of any one of paras 70-79, wherein the second end of the delivery system is coupled to a ventilator.
81. The system of any one of paras 70-80, wherein the system includes a pressure vessel containing a pressurized gas including at least about 1% nitric oxide.
82. A method for delivering nitric oxide, comprising:
   releasing a gas flow including at least 1% nitric oxide from a pressure vessel into a delivery system, wherein a first end of the delivery system is coupled to the pressure vessel and the delivery system is configured to communicate the gas flow to a second end of the delivery system;
   providing an inert gas into the delivery system from an inert gas source;
   diluting the gas flow with the inert gas in the delivery system; and
   passing the gas flow out of the second end of the delivery system.
83. The method of para 82, wherein the inert gas is nitrogen.
84. The method of para 82 or 83, wherein the delivery system is free from electronics.
85. The method of any one of paras 82-84, wherein the delivery system is free of a heating device.
86. The method of any one of paras 82-85, wherein the method includes regulating an amount of the gas flow released from a pressure vessel.
87. The method of para 86, wherein the amount of gas flow released is regulated by a gas regulator, which is coupled to the pressure vessel.
88. The method of para 86 or 87, wherein the amount of gas flow released is regulated by a restrictor, which is coupled to the pressure vessel.
89. The method of para 88, wherein the restrictor includes an orifice.
90. The method of para 88 or 89, wherein the restrictor includes a capillary tube.
91. The method of any one of paras 82-90, wherein the nitric oxide is suitable for inhalation by a mammal.
92. The method of any one of paras 82-91, wherein a patient interface is coupled to the second end of the delivery system.
93. The method of any one of paras 82-92, wherein a ventilator is coupled to the second end of the delivery system.
94. The method of any one of paras 82-93, wherein the method includes delivering nitric oxide to a patient.
95. The method of para 94, wherein the nitric oxide delivered to the patient is at a concentration of at least about 1 ppm.
96. The method of para 94 or 95, wherein the nitric oxide delivered to the patient is at a concentration of at least about 5 ppm.
97. The method of any one of paras 94-96, wherein the nitric oxide delivered to the patient is at a concentration of at least about 10 ppm.
98. The method of any one of paras 94-97, wherein the nitric oxide delivered to the patient is at a concentration of at least about 15 ppm.
99. The method of any one of paras 94-98, wherein the nitric oxide delivered to the patient is at a concentration of at least about 20 ppm.
100. The method of any one of paras 82-99, wherein the gas flow released from the pressure vessel includes at least about 1% nitric oxide.
101. The method of any one of paras 82-100, wherein the gas flow released from the pressure vessel includes at least about 5% nitric oxide.
102. The method of any one of paras 82-101, wherein the gas flow released from the pressure vessel includes at least about 25% nitric oxide.
103. The method of any one of paras 82-102, wherein the gas flow released from the pressure vessel includes at least about 50% nitric oxide.
104. The method of any one of paras 82-103, wherein the gas flow released from the pressure vessel includes at least about 75% nitric oxide.
105. The method of any one of paras 82-104, wherein the gas flow released from the pressure vessel includes at least about 90% nitric oxide.
106. The method of any one of paras 82-105, wherein the gas flow released from the pressure vessel includes at least about 4% and at most about 100% nitric oxide.
107. The method of any one of paras 82-106, wherein the gas flow released from the pressure vessel includes at least one inert gas.
108. The method of any one of paras 82-107, wherein the gas flow released from the pressure vessel includes nitrogen.
109. The method of any one of paras 82-108, wherein the gas flow released from the pressure vessel consists essentially of nitric oxide.
110. The method of any one of paras 82-109, wherein the pressure vessel holds a volume of at most about 10 mL.
111. The method of any one of paras 82-110, wherein the pressure vessel holds a volume of at most about 5 mL.
112. The method of any one of paras, 82-111, wherein the pressure vessel holds a volume of at most about 3 mL.

## Claims

1. A delivery system, comprising:
a first end configured to be coupled to a pressure vessel including a pressurized gas, wherein the pressurized gas includes at least 1% nitric oxide;
a second end, wherein the delivery system is configured to communicate a gas flow including nitric oxide to a second end of the delivery system; and
a coupler, wherein the coupler is configured to couple to an air source to provide air into the delivery system, such that air from the air source mixes in the delivery system with the gas in the gas flow resulting in a gas flow including at least 0.01% nitrogen dioxide; and
a reducing agent between the coupler and the second end, wherein the reducing agent reacts with nitrogen dioxide in the gas flow and converts the nitrogen dioxide to nitric oxide.

2. The system of claim 1, comprising an air source configured to provide air into the delivery system, such that air from the air source mixes in the delivery system with the gas in the gas flow resulting in a gas flow including at least 0.01% nitrogen dioxide.

3. The system of claim 1 or 2, wherein the reducing agent is included on a surface active material, preferably wherein the reducing agent is coated on a surface active material.

4. The system of any one of claims 1-3, wherein the surface active material is in a cartridge configured to receive the gas flow including nitrogen dioxide via an inlet and to communicate the gas flow including nitrogen dioxide through the surface-active material to an outlet.

5. The system of any one of claims 1-4, wherein the delivery system is free of electronics and/or a heating device.

6. The system of any one of claims 1-5, wherein the delivery system includes a gas regulator coupled to the storage device.

7. The system of any one of claims 1-6, wherein the delivery system includes a restrictor, preferably wherein the restrictor includes an orifice.

8. The system of claim 7, wherein the restrictor includes a capillary tube.

9. The system of any one of claims 1-8, wherein the second end of the delivery system is coupled to a patient interface and/or a ventilator.

10. The system of any one of claims 1-9, wherein the system includes a pressure vessel containing a pressurized gas including at least 1% nitric oxide.

11. A method for delivering nitric oxide, comprising:
releasing a gas flow including nitric oxide from a pressure vessel into a delivery system according to any one of claims 1 to 10;
providing air into the delivery system from an air source;
mixing the gas flow with the air in the delivery system such that, subsequent to mixing, the gas flow includes at least 0.01% nitrogen dioxide;
converting the nitrogen dioxide in the gas flow into nitric oxide; and
passing the gas flow out of the second end of the delivery system.

12. The method of claim 11, wherein converting the nitrogen dioxide in the gas flow into nitric oxide includes contacting the gas flow with a reducing agent, wherein the reducing agent reacts with nitrogen dioxide in the gas flow and converts the nitrogen dioxide to nitric oxide.

13. The method of any one of claims 11 or 12, wherein the method includes regulating an amount of the gas flow released from a pressure vessel, optionally wherein the amount of gas flow released is regulated by a gas regulator or a restrictor, which is coupled to the pressure vessel.

14. The method of any one of claims 11-13, wherein the gas flow released from the pressure vessel includes at least 1%, at least 5%, at least 25%, at least 50%, at least 75%, at least 90% nitric oxide, or at least 4% and at most 100% nitric oxide.

15. The method of any one of claims 11-14, wherein the gas flow released from the pressure vessel includes one or more inert gases, preferably nitrogen.

16. The method of any one of claims 11-15, wherein the gas flow released from the pressure vessel consists essentially of nitric oxide.

17. The method of any one of claims 11-16, wherein the pressure vessel holds a volume of at most 10 mL, at most 5 mL, or at most 3 mL.

18. A method of storing nitric oxide, comprising filling a pressure vessel with a pressurized gas including nitric oxide, wherein the pressurized gas is suitable for inhalation by a mammal, and wherein the pressure vessel holds volume of less than 5 L, the pressurized gas is stored at a pressure of at least 1500 psi and the pressurized gas has a volume of at most 800 L at standard temperature and pressure.
